# FASCICULE DE BREVET EUROPEEN

(11) **EP 1 216 022 B1**
(45) Date de publication et mention de la délivrance du brevet: **19.04.2006**
(21) Numéro de dépôt: 99946238.5
(22) Date de dépôt: 27.09.1999
(51) Int. Cl.: A61Q 5/06, A61K 8/892, A61K 8/893, A61K 8/898, A61K 8/899, A61K 8/897

(54) **COMPOSITION COSMETIQUE A BASE DE COMPOSES ORGANIQUES DU SILICIUM COMPORTANT AU MOINS UNE FONCTION SOLUBILISANTE NON-BASIQUE**
KOSMETISCHE ZUSAMMENSETZUNG AUF BASIS VON ORGANO-SILIZIUM VERBINDUNGEN, DIE MINDESTENS EINE LÖSLICH MACHENDE NICHT-BASISCHEN GRUPPE TRAGEN
COSMETIC COMPOSITIONS BASED ON ORGANIC SILICON COMPOUNDS COMPRISING AT LEAST ONE NON-BASIC SOLUBILISING FUNCTION

(43) Date de publication de la demande: 26.06.2002
(73) Titulaire: L'OREAL, 75008 Paris (FR)
(72) Inventeur: SAMAIN, Henri, F-91570 Bièvres (FR); ROLLAT-CORVOL, Isabelle, F-75017 Paris (FR); JEANNE ROSE, Valérie, F-75019 Paris (FR); SANCHEZ, Clément, F-91190 Gif-sur-Yvette (FR)
(74) Mandataire: Casalonga, Axel
(86) Numéro de dépôt international: PCT/FR1999/002290
(87) Numéro de publication internationale: WO 2001/022931

(56) Documents cités:
- EP-A- 0 242 855
- EP-A- 0 464 835
- EP-A- 1 023 889
- FR-A- 2 746 008
- US-A- 3 914 416

## Description

La présente invention concerne d'une manière générale des compositions cosmétiques aqueuses, en particulier pour le traitement des cheveux, comportant des composés organiques du silicium solubles dans l'eau, peu ou pas polymérisés.

Il est habituel d'utiliser des composés organiques tels que des polymères pour réaliser des compositions cosmétiques pour le traitement des cheveux. Par exemple, on utilise des polymères donnant au sèchage des matériaux solides pour fixer dans une forme la coiffure. De tels matériaux sont également utilisés pour donner des effets de maintien de la forme. On utilise aussi des composés polymériques, tels que des polysiloxanes, pour donner des effets de soin aux cheveux, particulièrement les cheveux abîmés ou difficiles à démêler. Les compositions cosmétiques contenant ces polymères sont déposées sur les cheveux qu'on laisse sécher ou que l'on rince avant de passer au séchage.

L'utilisation de composés polymériques présente plusieurs inconvénients.

Le premier inconvénient réside dans le fait que, lorsque les polymères sont utilisés dans des compositions au-delà d'une certaine concentration, les compositions obtenues s'appliquent difficilement du fait de l'accroissement de la viscosité de la composition. Cette difficulté d'application des compositions entraîne des surcharges en certains endroits de la chevelure et donc des défauts cosmétiques et implique également que certaines parties de la chevelure reçoivent moins de compositions, ce qui, au final, induit sur ces parties un moindre effet.

Le second inconvénient réside dans le fait que ces compositions sont parfois difficiles à mettre en oeuvre. En effet, les composés polymériques à faible solubilité dans l'eau, exigent l'utilisation de solvant organique ou de mélange de solvants organiques. L'emploi de solvant organique entraîne plusieurs problèmes, comme des problèmes d'environnement et d'effet sur la cosméticité des cheveux.

Pour remédier à ces inconvénients, on s'est donc tourné vers l'utilisation de composés polymériques rendus partiellement solubles dans l'eau. Ainsi, certains composés polymériques peuvent être utilisés dans l'eau sans ajout d'un quelconque co-solvant. Dans ce cas, la limitation réside dans le fait que ces composés polymériques sont éliminés partiellement, voire totalement, par rinçage des cheveux. Par conséquent, dans ce cas, l'effet dû aux composés polymériques est très limité après rinçage. Au final, cela limite l'effet des traitements rincés (shampooing, après shampooing), mais réduit aussi l'intérêt de telles compositions utilisées en mode non rincé (laques, mousses, lotions de mise en plis, etc.) dans la mesure où l'utilisateur perd l'effet acquis par le traitement lorsqu'il se lave les cheveux.

Des efforts ont donc été réalisés pour trouver des composés pour la formulation de compositions cosmétiques qui soient utilisables dans l'eau et qui présentent une rémanence de leur effet lorsque les cheveux sont rincés.

Ainsi, le brevet des Etats-Unis n° 4 344 763 (GILLETTE) décrit des compositions cosmétiques comportant un monomère organosiloxane tel qu'un aminoalkylalcoxysilane et un titanate organique en solution dans un alcool.

Plus précisément, ce brevet décrit un procédé de mise en forme des cheveux qui consiste à les humidifier avec de l'eau puis à appliquer une solution contenant dans de l'isopropanol de 0,5 à 15% en poids d'un aminoalkylalcoxysilane et de 0,005 à 1,5% en poids d'un titanate organique et à mettre ensuite les cheveux dans la forme souhaitée.

Selon ce procédé, il est particulièrement recommandé de maintenir la solution d'isopropanol à l'abri de toute humidité.

Il a également été décrit dans le brevet EP-113 992, un procédé pour simultanément fixer et conditionner les cheveux à l'aide d'une composition, stable en l'absence d'humidité, contenant (A) un oligomère de siloxane ayant au moins une liaison azote-hydrogène, et (B) un additif anhydre, facilement hydrolysable, choisis parmi les titanates, zirconates, vanadates, germanates, et leurs mélanges.

Le solvant de la composition est un hydrocarbure aliphatique ou un halogénohydrocarbure aliphatique, de préférence le 1,1,1-trichloroéthane.

Après application de la composition sur les cheveux, ceux-ci sont placés en atmosphère humide afin de provoquer la réticulation de l'oligomère de siloxane et de l'additif anhydre facilement hydrolysable.

EP-0 464 835 utilise des silanes organofonctionels pre-hydrolysés comme agents fixants des cheveux faciles à éliminer au lavage.

Il existe donc un besoin d'une composition cosmétique stable, en particulier pour le traitement des cheveux, qui soit essentiellement aqueuse et qui permette d'obtenir un effet cosmétique suffisant, en particulier pour les cheveux en mode rincé ou non rincé.

La présente invention a donc pour objet des compositions cosmétiques aqueuses, stables, en particulier des compositions cosmétiques pour le traitement et le soin des cheveux, qui remédient aux inconvénients de l'art antérieur.

Plus précisément, la présente invention a pour objet des compositions cosmétiques aqueuses, stables, pour le traitement et le soin des cheveux, conférant aux cheveux un effet coiffant de longue durée et un toucher agréable.

Le Demandeur a remarqué, de façon surprenante, qu'il était possible de formuler des compositions cosmétiques ne nécessitant pas l'utilisation de solvant organique et qui présentaient un effet cosmétique efficace, résistant au rinçage, sans risque de problèmes de cheveux chargés en cas de superposition, en utilisant dans ces compositions des composés organiques du silicium peu ou pas polymérisés, solubles dans l'eau, comportant au moins une fonction chimique solubilisante non basique.

On a observé que l'application de telles compositions permet d'obtenir des effets cosmétiques marqués, sans problème en cas de superposition, dont les effets résistent bien au rinçage et au lavage.

Selon l'invention, les compositions cosmétiques, en particulier pour le traitement des cheveux, comprennent, dans un milieu aqueux cosmétiquement acceptable, au moins 0,5% en poids par rapport au poids total de la composition, d'un ou plusieurs composés organiques du silicium solubles dans l'eau, peu ou pas polymérisés, choisis parmi les organosilanes comportant un atome de silicium et les organosiloxanes comportant deux ou trois atomes de silicium, les composés organiques du silicium comportant en outre au moins une fonction chimique solubilisante non basique et au moins deux groupes hydrolysables par molécule, selon la revendicalion 1.

Les composés organiques du silicium selon l'invention sont susceptibles de former, en milieu aqueux, un composé non hybride, après condensation sur eux-mêmes et évaporation du support. On entend par composé non hybride, un composé chimiquement homogène quant au silicium, c'est-à-dire qu'il ne renferme pas d'autres espèces métalliques ou organométalliques supplémentaires.

Les composés organiques du silicium, peu ou pas polymérisés, utiles dans les compositions de la présente invention sont choisis parmi les organosilanes solubles dans l'eau, comprenant un atome de silicium et les organosiloxanes solubles dans l'eau, comportant deux ou trois atomes de silicium, de préférence deux. Ils doivent en outre comporter au moins une fonction chimique solubilisante non basique, et de préférence une seule fonction. Parmi les fonctions solubilisantes non basiques, les acides carboxyliques et leurs sels, les acides sulfoniques et leurs sels et les restes poly(alkyléthers) tels que les restes poly(oxyalkylènes), par exemple poly(oxyde d'éthylène) et poly(oxyde de propylène) et les polyglycols, les polyacrylamides et acrylamides, sont conformes à l'invention.

Les composés organiques du silicium utiles dans les compositions de la présente invention, comportent en outre au moins deux groupes hydrolysables ou hydroxyles par atome de silicium. Les groupes hydrolysables sont des groupes alcoxy, aryloxy ou halogènes. Ils peuvent également, éventuellement, comporter d'autres fonctions chimiques telles que des fonctions acides ou amines.

Lorsque le composé de silicium peu ou pas polymérisé, selon l'invention, comporte un groupe non hydrolysable, ce groupe peut également comporter une fonction chimique telle qu'une fonction acide ou amine.

Les organosilanes préférés selon l'invention répondent à la formule : 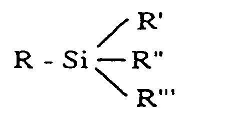 dans laquelle :
R' représente un halogène, un groupe OR₁ ou R_{O};
R" représente un halogène, un groupe OR₂ ou R'_{O};
R"' représente un halogène, un groupe OR₃ ou R"_{O};
deux au moins des groupes R', R" et R"' étant différents des groupes R_{O}, R'_{O} et R"_{O} ;
R est un groupe hydrocarboné, saturé ou insaturé, linéaire ou ramifié, comportant une fonction chimique solubilisante non basique, en particulier non aminée;
R₁ R₂, R₃, R_{O}, R'_{O} et R"_{O} représentent, indépendamment les uns des autres, un groupe hydrocarboné saturé ou insaturé, linéaire ou ramifié, portant éventuellement des groupes chimiques supplémentaires solubilisants non-basiques, R₁, R₂ et R₃ pouvant en outre désigner l'hydrogène.

De préférence, R₁, R₂, R₃, R_{O}, R'_{O} et R"_{O} représentent un groupe alkyle de C₁ à C₁₂, un groupe aryle de C₆ à C₁₄, un groupe alkyle de C₁ à C₈₋aryle de C₆ à C₁₄, et un groupe aryle de C₆ à C₁₄-alkyle C₁ à C₈.

Les organosiloxanes préférés dans les compositions de la présente invention peuvent être représentés par la formule : 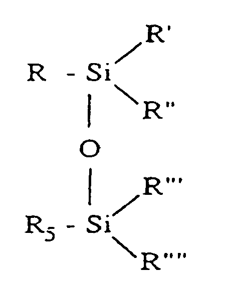 dans laquelle :
R" représente un halogène ou un groupe OR₂;
R, R₂, R' et R"' sont définis comme précédemment, R"" représente un halogène, un groupe OR₄ ou un groupe R"'_{O};
R₅ représente un halogène, un groupe OR₆ ou un groupe R""_{O};
R₄, R₆, R"'_{O} et R""_{O} représentent un groupe hydrocarboné, saturé ou insaturé, linéaire ou ramifié, portant éventuellement des groupes chimiques supplémentaires solubilisants non basiques, R₄ et R₆ pouvant en outre désigner l'hydrogène; l'un au moins des groupes R''', R"" et R₅ étant différent de R"_{O}, R"'_{O} et R""_{O}.

De préférence R₄, R₆, R_{O}, R"_{O}, R"'_{O} et R""_{O} représentent un groupe alkyle de C₁ à C₁₂, un groupe aryle de C₆ à C₁₄, un groupe alkyle de C₁ à C₈-aryle de C₆ à C₁₄, et un groupe aryle de C₆ à C₁₄-alkyle de C₁ à C₈; et R₅ représente de préférence un groupe alkyle de C₁ à C₁₂, un groupe alcoxy de C₁ à C₁₂, un groupe aryle de C₆ à C₁₄, un groupe alkyle de C₁ à C₈-aryle de C₆ à C₁₄, et un groupe aryle de C₆ à C₁₄-alkyle de C₁ à C₈.

De préférence, l'halogène est le chlore.

Les groupes R sont choisis de préférence parmi les acides carboxyliques et leurs sels, les ammonium quaternaires, les acides sulfoniques et leurs sels, et les polyalkyléthers.

Parmi les fonctions acides carboxyliques et leurs sels, on peut citer les radicaux des monoacides saturés tels que les acides acétique, propionique, butyrique, isobutyrique, valérique, isovalérique, les diacides saturés tels que les acides oxalique, malonique, succinique, glutarique et adipique, les monoacides insaturés tels que l'acide acrylique, l'acide méthacrylique, l'acide crotonique, l'acide maléique, l'acide fumarique et l'acide citraconique, les acides carbocycliques tels que l'acide benzoïque, l'acide phtalique, l'acide isophtalique, l'acide térèphtalique, les hydroxy et alcoxy-acides carboxyliques tels que l'acide glycolique, l'acide lactique, l'acide tartrique et l'acide salicylique et les sels de ces acides, en particulier les sels alcalins et plus particulièrement les sels de sodium et de potassium de ces acides.

Parmi les fonctions ammonium quaternaires, on peut citer les tétraalkylammonium, les alkylarylammonium quaternaires, les groupements alkyle et/ou aryle pouvant éventuellement comporter des fonctions telles que des fonctions acides, hydroxyles, amines et halogènes, et les ammonium quaternaires cycliques et hétérocycliques.

Parmi les acides sulfoniques et leurs sels, on peut citer les acides alkylsulfoniques tels que l'acide méthylsulfonique, les acides arylsulfoniques tels que l'acide phénylsulfonique, les acides alcoxy-sulfoniques tels que l'acide éthoxysulfonique, les acides alkylaryl et arylalkylsulfoniques, et les sels de ces acides, en particulier les sels alcalins de ces acides et plus particulièrement les sels de sodium et de potassium de ces acides.

Parmi les restes alkyléthers, on peut citer les poly(oxyéthylène), les poly(oxypropylène), les poly(oxytétraméthylène) et les polyglycols tels que le poly(éthylèneglycol) et le poly(propylèneglycol).

Un autre aspect important des compositions selon l'invention est qu'elles contiennent des quantités importantes des composés organiques du silicium peu ou pas polymérisés, c'est-à-dire comportant un, deux ou trois atomes de silicium. Ainsi, il est nécessaire que la composition contienne, par rapport au poids total de la composition, au moins 0,02% de composés organiques du silicium peu ou pas polymérisés, et de préférence au moins 0,5% en poids et pouvant aller jusqu'à 50% en poids.

Le taux des composés organiques du silicium peu ou pas polymérisés, selon l'invention, est déterminé par des méthodes habituelles d'analyse telles que la spectroscopie RMN du silicium 29 et du proton, et par chromatographie.

Les compositions selon l'invention sont des compositions aqueuses. Toutefois, il est possible, pour la mise en oeuvre d'adjuvants par exemple, d'ajouter un co-solvant tel qu'un alcool ou une cétone, par exemple l'alcool éthylique ou l'acétone.

De façon connue, toutes les compositions de l'invention peuvent contenir les adjuvants habituels dans le domaine cosmétique, tels que des huiles, cires ou autres corps gras usuels; des gélifiants et/ou épaississants classiques; des émulsionnants; des agents hydratants; des émollients, des filtres solaires; des actifs hydrophiles ou lipophiles comme des céramides; des agents anti-radicaux libres; des tensio-actifs; des polymères; des protéines; des bactéricides; des séquestrants; des anti-pelliculaires; des anti-oxydants; des conservateurs; des parfums; des charges; des matières colorantes.

Les quantités de ces différents adjuvants sont celles classiquement utilisées dans le domaine considéré.

Bien entendu, l'homme de l'art veillera à choisir le ou les éventuels composés ajoutés à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont utilisables en mode rincé ou non rincé.

Les compositions selon l'invention peuvent se présenter sous toutes les formes appropriées pour une application topique, notamment sous forme de solutions du type lotions ou sérum; sous forme de gels aqueux; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses.

Ces compositions sont préparées selon les méthodes usuelles.

Les compositions selon l'invention sont de préférence utilisées comme produits capillaires, notamment pour le maintien de la coiffure ou de la mise en forme des cheveux. Elles peuvent en outre apporter de la coloration temporaire aux cheveux, bien assurer la protection des cheveux contre les effets des radiations UV, tout en apportant des propriétés de maintien ou de fixation des cheveux.

Les compositions capillaires, selon l'invention, sont de préférence des produits de coiffage tels que des gels, des lotions de mises en pli, des lotions pour le brushing, les compositions de fixation et de coiffage telles que des laques ou spray.

Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsqu'on souhaite obtenir un spray, une mousse pour la fixation ou le traitement des cheveux.

La présente invention a également pour objet l'utilisation de la composition selon l'invention dans un procédé de traitement des cheveux, en vue de leur maintien et/ou coloration.

Selon un mode de réalisation de ce procédé, on applique la composition sur les cheveux rincés ou non, de préférence sous la forme d'un spray, soit à l'aide d'un flacon pompe, soit à l'aide d'un aérosol.

Après pulvérisation sur l'ensemble de la chevelure, on laisse agir et sécher la composition.

Les cheveux peuvent être mis dans la forme souhaitée, soit avant l'application, soit immédiatement après.

Le temps de séchage peut être variable et est fonction de la nature de la composition.

Les cheveux, après peignage, présentent une qualité de toucher très agréable.

L'invention est illustrée par les exemples suivants :

### EXEMPLE 1

On a réalisé la formulation suivante :

| Composition | Composé du silicium peu ou pas polymérisé, soluble dans l'eau | Eau |
|---|---|---|
| | N-[(3-triméthoxysilyl)propyl] -éthylènediaminetriacétate de sodium neutralisé par HCl (pH 6,9) (fourni par la Société GELEST) (g pour 100 g de composition) | |
| 1 | 10 g (Matière active) | qsp 100 g |

La composition 1 est introduite dans un récipient que l'on munit d'un système de pulvérisation du type flacon pompe.

On prépare deux perruques de 15 g de cheveux naturels. Les cheveux sont maintenus aux racines sur bande de caoutchouc et laissés libres sur le reste de la longueur.

La composition est pulvérisée sur la première perruque (2 g). Cette perruque est laissée ainsi jusqu'à séchage. L'autre perruque, servant de comparatif, est laissée aussi au repos.

On note les propriétés cosmétiques et en particulier, l'effet coiffant.

Les cheveux sont alors lavés avec un shampooing au lauryléther sulfate de sodium, puis resséchés.

On note une seconde fois les propriétés cosmétiques.

| Perruque | Effet coiffant avant lavage | Effet coiffant après lavage |
|---|---|---|
| 1 | 30 | 15 |
| 2 (comparatif) | 0 | 0 |

Les résultats montrent que l'application d'une composition selon l'invention sur des cheveux, leur confère un effet coiffant résistant au lavage.

## Revendications

1. Composition cosmétique comprenant, dans un milieu aqueux cosmétiquement acceptable, au moins 0,5% en poids par rapport au poids total de la composition d'un ou plusieurs composés organiques du silicium soluble dans l'eau, peu ou pas polymérisés, choisis parmi les silanes comprenant un atome de silicium et comportant en outre au moins une fonction chimique solubilisante non-basique choisie parmi les acides carboxyliques et leurs sels, les acides sulfoniques et leurs sels, les poly(alkyléthers), les polyacrylamides et acrylamides, et au moins deux groupes hydroxyles, alcoxy, aryloxy ou halogènes, pas molécule, et les siloxanes comprenant deux ou trois atomes de silicium et comportant en outre au moins une fonction chimique solubilisante non-basique choisie parmi les acides carboxyliques et leurs sels, les ammonium quaternaires, les acides sulfoniques et leurs sels, les poly(alkyléthers), les polyacrylamides et acrylamides et les polyols, et au moins deux groupes hydroxyles ou hydrolysables par molécule.

2. Composition selon revendication 1, **caractérisée en ce que** les groupes hydrolysables sont choisis parmi les groupes alcoxy, aryloxy et halogènes.

3. Composition cosmétique selon la revendication 1 ou 2, **caractérisée en ce que** le ou les composés organiques du silicium, peu ou pas polymérisés, sont choisis parmi les composés de formules : 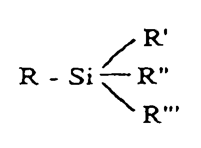 dans laquelle :
R' représente un halogène, un groupe OR₁ ou R_{O};
R" représente un halogène, un groupe OR₂ ou R'_{O};
R'" représente un halogène, un groupe OR₃ ou R"_{O};
deux au moins des groupes R', R" et R'" étant différents des groupes R_{O}, R'_{O} et R"_{O};
R est un groupe hydrocarboné, saturé ou insaturé, linéaire ou ramifié, comportant une fonction chimique solubilisante non basique choisie parmi les acides carboxyliques et leurs sels, les acides sulfoniques et leurs sels, les poly(alkyléthers), les polyacrylamides et acrylamides,
R_{O}, R'_{O}, R"_{O}, R₁, R₂ et R₃ représentent, indépendamment les uns des autres, un groupe hydrocarboné saturé ou insaturé, linéaire ou ramifié, portant éventuellement des groupes chimiques supplémentaires solubilisants non-basiques choisis parmi les acides carboxyliques et leurs sels, les acides sulfoniques et leurs sels, les poly(alkyléthers), les polyacrylamides et acrylamides, R₁, R₂ et R₃ pouvant en outre désigner l'hydrogène; et 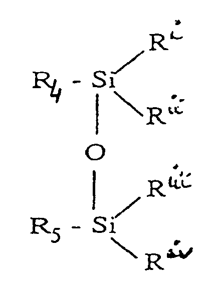
dans laquelle :
R₄ est un groupe hydrocarboné, saturé ou insaturé, linéaire ou ramifié, comportant une fonction chimique solubilisante non basique choisie parmi les acides carboxyliques et leurs sels, les ammoniums quaternaires, les acides sulfoniques et leurs sels, les poly(alkyléthers), les polyacrylamides et acrylamides et les polyols ;
Rⁱ représente un halogène, un groupe OR₁' ou Rₚ ;
Rⁱⁱ représente un halogène ou un groupe OR₂' ;
Rⁱⁱⁱ représente un halogène, un groupe OR₃' ou Rₚ" ;
R^{iv} représente un halogène, un groupe OR₄' ou Rₚ"' ;
R₅ représente un halogène, un groupe OR₅' ou R""ₚ;
l'un au moins des groupes Rⁱⁱⁱ, R^{iv}et R₅ étant différent de R"ₚ, R"'ₚ et R""ₚ; et
les groupes R₁', R₂', R₃', R₄', R₅', Rₚ, Rₚ", R"'ₚ et R""ₚ représentent indépendamment les uns des autres, un groupe hydrocarboné saturé ou insaturé, linéaire ou ramifié, portant éventuellement des groupes chimiques, solubilisants non basiques supplémentaires choisis parmi les acides carboxyliques et leurs sels, les ammoniums quaternaires, les acides sulfoniques et leurs sels, les poly(alkyléthers), les polyacrylamides et acrylamides et les polyols, R₁', R₂', R₃', R₄' et R₅' pouvant en outre désigner l'hydrogène.

4. Composition cosmétique selon la revendication 3, **caractérisée en ce que** R₁, R₂, R₃, R'₁, R'₂, R'₃, R'₄ R₅' R_{O}, R'_{O}, R''_{O}, Rₚ, R'ₚ, R"ₚ, R"'ₚ et R""ₚ représentent un groupe alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

5. Utilisation de la composition selon l'une quelconque des revendications précédentes comme produit capillaire.

6. Utilisation selon la revendication 5, pour le maintien de la coiffure ou la mise en forme des cheveux.

## Patentansprüche

1. Kosmetische Zusammensetzung, die in einem kosmetisch akzeptablen, wässerigen Medium, bezogen auf das Gesamtgewicht der Zusammensetzung, mindestens 0,5 Gew.-% einer oder mehrerer wenig oder nicht polymerisierter, in Wasser löslicher, Siliciumorganischer Verbindungen enthält, die unter den Silanen, die ein Siliciumatom, ferner mindestens eine nicht basische, solubilisierende chemische Funktion, die unter den Carbonsäuren und ihren Salzen, den Sulfonsäuren und ihren Salzen, Poly(alkylethern), Polyacrylamiden und Acrylamiden ausgewählt ist, und mindestens zwei Hydroxygruppen, Alkoxygruppen, Aryloxygruppen oder Halogene pro Molekül enthalten,
und den Siloxanen ausgewählt sind, die zwei oder drei Siliciumatome, ferner mindestens eine nicht basische, solubilisierende chemische Funktion, die unter den Carbonsäuren und ihren Salzen, quartären Ammoniumverbindungen, Sulfonsäuren und ihren Salzen, Poly(alkylethern), Polyacrylamiden, Acrylamiden und Polyolen ausgewählt ist, und mindestens zwei Hydroxygruppen oder hydrolysierbare Gruppen pro Molekül enthalten.

2. Zusammensetzung nach Anspruch 1, **dadurch gekennzeichnet, dass** die hydrolysierbaren Gruppen unter den Alkoxygruppen, Aryloxygruppen und Halogenen ausgewählt sind.

3. Kosmetische Zusammensetzung nach Anspruch 1 oder 2, **dadurch gekennzeichnet, dass** die wenig oder nichtpolymerisierbare(n) Silicium-organische(n) Verbindung(en) unter den Verbindungen der folgenden Formel ausgewählt sind: 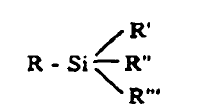 worin bedeuten:
R' Halogen, OR₁ oder Rₒ;
R" ein Halogen, OR₂ oder R'ₒ;
R'" ein Halogen, OR₃ oder R"o;
wobei mindestens zwei der Gruppen R', R" und R'" von den Gruppen Rₒ, R'ₒ und R"ₒ verschieden sind;
R eine gesättigte oder ungesättigte, geradkettige oder verzweigte Kohlenwasserstoffgruppe, die eine nicht basische, solubilisierende chemische Funktion besitzt, die unter den Carbonsäuren und ihren Salzen, den Sulfonsäuren und ihren Salzen, Polyalkylethern, Polyacrylamiden und Acrylamiden ausgewählt ist;
Rₒ, R'ₒ, R"ₒ, R₁, R₂ und R₃ unabhängig voneinander eine gesättigte oder ungesättigte, geradkettige oder verzweigte Kohlenwasserstoffgruppe, die gegebenenfalls ergänzende, nicht basische, solubilisierende chemische Gruppen aufweist, die unter den Carbonsäuren und ihren Salzen, Sulfonsäuren und ihren Salzen, Polyalkylethern, Polyacrylamiden und Acrylamiden ausgewählt ist, wobei R₁, R₂ und R₃ ferner Wasserstoff bedeuten können; und 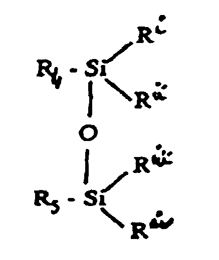
worin bedeuten:
R₄ eine gesättigte oder ungesättigte, geradkettige oder verzweigte Kohlenwasserstoffgruppe, die eine nicht basische, solubilisierende chemische Funktion aufweist, die unter den Carbonsäuren und ihren Salzen, quartären Ammoniumverbindungen, Sulfonsäuren und ihren Salzen Poly(alkylethern), Polyacrylamiden, Acrylamiden und Polyolen ausgewählt ist; Rⁱ ein Halogen, OR₁' oder Rₚ;
Rⁱⁱ ein Halogen oder OR₂';
Rⁱⁱⁱ ein Halogen, OR₃' oder Rₚ";
R^{iv} ein Halogen, OR₄' oder Rₚ'";
R₅ ein Halogen, OR₅' oder R""ₚ;
wobei mindestens eine der Gruppen Rⁱⁱⁱ, R^{iv} und R₅ von R"ₚ, R"'ₚ und R""ₚ verschieden ist; und
die Gruppen R₁', R₂', R₃', R₄', R₅', Rₚ, Rₚ", R"'ₚ und R""ₚ unabhängig voneinander eine gesättigte oder ungesättigte, geradkettige oder verzweigte Kohlenwasserstoffgruppe, die gegebenenfalls ergänzende, nicht basische, solubilisierende chemische Gruppen trägt, die unter den Carbonsäuren und ihren Salzen, quartären Ammoniumverbindungen, Sulfonsäuren und ihren Salzen, Poly(alkylethern), Polyacrylamiden und Acrylamiden und Polyolen ausgewählt sind, wobei R₁', R₂', R₃', R₄' und R₅' ferner Wasserstoff bedeuten können.

4. Kosmetische Zusammensetzung nach Anspruch 3, **dadurch gekennzeichnet, dass** R₁, R₂, R₃, R'₁, R'₂, R'₃, R'₄, R'₅, Rₒ, R'ₒ, R"ₒ, Rₚ, R'ₚ, R"ₚ, R"'ₚ und R""ₚ C₁₋₁₂-Alkyl, C₆₋₁₄-Aryl, C₁₋₈-Alkyl-C₆₋₁₄₋aryl und C₆₋₁₄-Aryl-C₁₋₈-alkyl bedeuten.

5. Verwendung der Zusammensetzung nach einem der vorhergehenden Ansprüche als Produkt für die Haarbehandlung.

6. Verwendung nach Anspruch 5 für die Festigung der Frisur oder die Formgebung der Frisur.

## Claims

1. Cosmetic composition comprising, in a cosmetically acceptable aqueous medium, at least 0.5% by weight, relative to the total weight of the composition, of one or more unpolymerized or relatively unpolymerized, water-soluble organosilicon compounds chosen from silanes comprising one silicon atom and also comprising at least one nonbasic solubilizing chemical function, chosen from carboxylic acids and salts thereof, sulfonic acids and salts thereof, poly(alkyl ethers), polyacrylamides and acrylamides,
and at least two hydroxyl, alkoxy, aryloxy or halogen groups per molecule,
and siloxanes comprising two or three silicon atoms and also comprising at least one nonbasic solubilizing chemical function chosen from carboxylic acids and salts thereof, quaternary ammoniums, sulfonic acids and salts thereof, poly(alkyl ethers), polyacrylamides and acrylamides, and polyols,
and at least two hydroxyl or hydrolysable groups per molecule.

2. Composition according to Claim 1, **characterized in that** the hydrolysable groups are chosen from alkoxy, aryloxy and halogen groups.

3. Cosmetic composition according to Claim 1 or 2, **characterized in that** the unpolymerized or relatively unpolymerized organosilicon compound(s) is (are) chosen from the compounds of formulae: 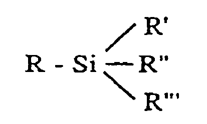 in which:
R' represents a halogen or a group OR₁ or Rₒ;
R" represents a halogen or a group OR₂ or R'ₒ;
R"' represents a halogen or a group OR₃ or R"ₒ;
at least two of the groups R', R" and R"' being other than the groups Rₒ, R'ₒ and R"ₒ;
R is a saturated or unsaturated, linear or branched hydrocarbon-based group comprising a nonbasic solubilizing chemical function chosen from carboxylic acids and salts thereof, sulfonic acids and salts thereof, poly(alkyl ethers), polyacrylamides and acrylamides;
Rₒ, R'ₒ, R"ₒ, R₁, R₂ and R₃ represent, independently of each other, a saturated or unsaturated, linear or branched hydrocarbon-based group optionally bearing additional nonbasic solubilizing chemical groups, chosen from carboxylic acids and salts thereof, sulfonic acids and salts thereof, poly(alkyl ethers), polyacrylamides and acrylamides, R₁, R₂ and R₃ also possibly denoting hydrogen; and 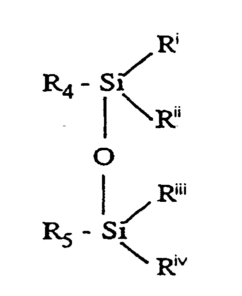
in which:
R₄ is a linear or branched, saturated or unsaturated hydrocarbon-based group comprising a nonbasic solubilizing chemical function chosen from carboxylic acids and salts thereof, quaternary ammoniums, sulfonic acids and salts thereof, poly(alkyl ethers), polyacrylamides and acrylamides, and polyols;
Rⁱ represents a halogen or a group OR₁' or Rₚ;
Rⁱⁱ represents a halogen or a group OR₂';
Rⁱⁱⁱ represents a halogen or a group OR₃ or Rₚ";
R^{iv} represents a halogen or a group OR₄' or Rₚ' ";
R₅ represents a halogen or a group OR₅' or R""ₚ;
at least one of the groups Rⁱⁱⁱ, R^{iv} and R₅ being other than R"ₚ, R"'ₚ and R""ₚ; and
the groups R₁' , R₂', R₃' , R₄' , R₅' , Rₚ, Rₚ" , Rₚ' " and
Rₚ"" represent, independently of each other, a linear or branched, saturated or unsaturated hydrocarbon-based group, optionally bearing additional nonbasic solubilizing chemical groups chosen from carboxylic acids and salts thereof, quaternary ammoniums, sulfonic acids and salts thereof, poly(alkyl ethers), polyacrylamides and acrylamides, and polyols, R₁', R₂', R₃', R₄' and R₅' also possibly denoting hydrogen.

4. Cosmetic composition according to Claim 3, **characterized in that** R₁, R₂, R₃, R'₁, R'₂, R'₃, R'₄, R'₅, Rₒ, R'ₒ, R"ₒ, Rₚ, R'ₚ, R"ₚ, R"'ₚ and R""ₚ represent a C₁-C₁₂ alkyl group, a C₆ to C₁₄ aryl group, a (C₁ to C₈) alkyl (C₆ to C₁₄) aryl group and a (C₆ to C₁₄)aryl(C₁ to C₈) alkyl group.

5. Use of the composition according to any one of the preceding claims, as a hair product.

6. Use according to Claim 5, for holding the hairstyle or for shaping the hair.
